# EUROPEAN PATENT SPECIFICATION

(11) **EP 2 618 853 B1**
(45) Date of publication and mention of the grant of the patent: **27.01.2016**
(21) Application number: 11746647.4
(22) Date of filing: 20.07.2011
(51) Int. Cl.: A61L 2/22, A61L 9/14

(54) **A ROOM SANITIZING APPARATUS**
RAUMDESINFEKTIONSVORRICHTUNG
APPAREIL DE DÉSINFECTION DE LOCAUX

(30) Priority: 22.07.2010 IT BO20100461
(43) Date of publication of application: 31.07.2013
(73) Proprietor: 99 Holding S.A.R.L, 1449 Luxembourg (LU)
(72) Inventor: MALAGUTI SIMONI, Gian Luca, 98000 Monaco (MC)
(74) Representative: Firmati, Leonardo
(86) International application number: PCT/IB2011/053230
(87) International publication number: WO 2012/011060

(56) References cited:
- WO-A1-03/082355
- WO-A1-2008/025168
- WO-A2-2008/144202
- FR-A1- 2 913 340
- US-A1- 2005 163 685
- US-A1- 2005 257 689
- US-A1- 2008 226 495

## Description

### Technical Field

This invention relates to a room sanitizing apparatus.

More specifically, this invention has for an object an apparatus comprising a nebulizing device and used for sanitizing rooms, as well as the walls delimiting the rooms and materials and equipment located in the rooms.

The invention also relates to a method for sanitizing rooms using a nebulizing device.

### Background Art

Room sanitizing apparatuses equipped with nebulizing devices have become widely used, especially for sanitizing/sterilizing operating rooms, delivery rooms, laboratories, doctors' surgeries, and the like. Document WO 2008/144202 A discloses an apparatus for disinfecting rooms by means of an aerosol.

In this specification, the expressions sanitizing and sanitizing treatment are used to mean any disinfection and/or decontamination treatment for germicidal and/or anti-bacterial and/or disinfection purposes, that is to say, intended to make the treated room as sterile as possible, and hence safe also for particularly weak or vulnerable people, such as sick persons, infants, etc.

The expression germicidal is used in a broad sense to mean any bactericidal, virucidal, sporicidal, fungicidal or biofilm destroying action.

Nebulizing devices of the above mentioned prior art type cause microparticles of germicidal/antibacterial liquid solution to be distributed by diffusion in the treated room, and to settle also on the surfaces that delimit the room and on the surfaces of any furnishings and equipment located inside the room. Preferably, the nebulizing device diffuses in the rooms to be sanitized a disinfectant aqueous solution, based on hydrogen peroxide, in the form of a dry mist.

By way of example, reference may be made to a hospital operating room and all the machinery and equipment it contains.

Since the substances used for the sanitizing treatment, precisely because of the action they must apply, may in some cases be highly toxic if present in the air breathed by the human occupants of the room, it is necessary to let a certain length of time pass after treatment so that the concentration of these substances is reduced below a certain level.

In other words, when the interior of a room is sanitized, it is isolated from the exterior and a certain amount of time must be allowed to pass before giving personnel access to the room when the airborne concentration of the potentially toxic sanitizing substances is down to an acceptable level again.

Since the time that must pass may be particularly long, even in the order several hours, it is evident that a sanitizing treatment makes the treated room unusable for an extended period of time, due to the time spent for the actual treatment added to the time that must be allowed to pass after treatment. This circumstance obviously has negative consequences on the running for example of an operating room because it cannot be used to the full but only for a few hours a day.

This factor, besides having serious economic consequences, also involves risks for patients in the event of emergency operations and, generally speaking, in all events, means poor utilization of resources.

It is quite obvious, for example, that if a hospital has to guarantee the availability of an operating room at all times, it must be provided with two or more substantially equivalent operating rooms so that at least one room is available when the others are out of bounds on account of sanitizing in progress or post-sanitization waiting time necessary for lowering the level of the sanitizing substances used for the treatment.

A further drawback connected with the use of prior art apparatuses is due to the fact that their moving parts (electric motors, impellers), as a result of wear, produce dust and airborne particles which are released into the room being sanitized.

### Aim of the Invention

This invention has for an aim to overcome the above mentioned drawbacks by providing a room sanitizing apparatus which is particularly efficient and at once inexpensive to make and practical and effective to use.

Another aim of the invention is to provide a room sanitizing apparatus that allows the airborne concentration of the sanitizing substances to be rapidly reduced.

The technical features of the invention according to the above mentioned aim may be easily inferred from the content of the appended claims, especially claim 1, and preferably any of the claims that depend, either directly or indirectly, on claim 1.

### Brief Description of the Drawings

The advantages of this invention are apparent from the detailed description which follows, with reference to the accompanying drawings which illustrate a preferred non-limiting embodiment of it and in which:
- Figure 1 is a schematic front elevation view illustrating a preferred embodiment of the room sanitizing apparatus made according to this invention;
- Figure 2 is a schematic side elevation view of the room sanitizing apparatus of Figure 1;
- Figure 3 is schematic view from the side indicated by the arrow III of Figure 2 of a detail of the apparatus illustrated in the preceding figures.

### Detailed Description of the Preferred Embodiments of the Invention

With reference to the accompanying drawings, which illustrate a preferred embodiment of the apparatus according to the invention, the numeral 1 denotes in its entirety a room sanitizing apparatus made in accordance with the invention.

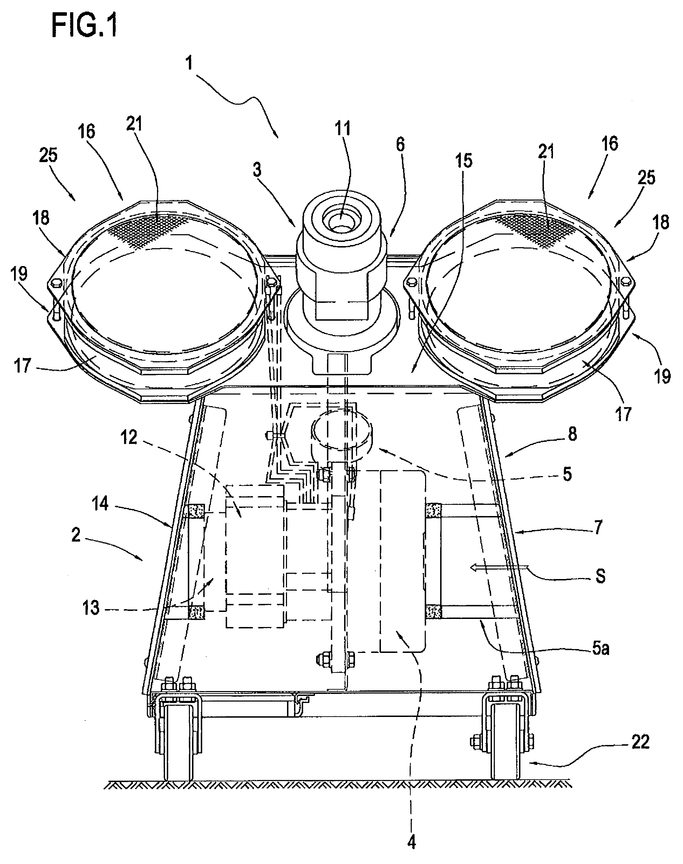

As illustrated in Figure 1, the room sanitizing apparatus 1 comprises a central structure 2 having the general shape of a truncated pyramid and housing a device 3 for nebulizing a liquid substances.

The nebulizing device 3 is of a substantially known type and will not therefore be described in detail in this specification, at least with regard to its known structural and/or functional aspects.

The central structure 2 defines a mounting frame for supporting and containing the nebulizing device 3.

The nebulizing device 3 comprises a blowing element 4 for taking air in from the room and directing it, with a due increase in its pressure, through a respective first circulation duct 5, to a Venturi effect device 6 located at the top of the structure 2 and rising up therefrom.

The first duct 5 comprises a first stretch 5a and a second stretch 5b, respectively upstream and downstream of the blowing element 4 relative to the direction of the air flow produced by the selfsame blowing element 4, indicated in Figure 1 by an arrow S.

The first stretch 5a of the first duct 5 extends from a grille 7 located on a first flank 8 of the structure 2 and designed to allow the room air to be taken in from the outside of it.

The second stretch 5b of the first duct 5, on the other hand, starts at the blowing element 4 and ends at the Venturi effect device 6.

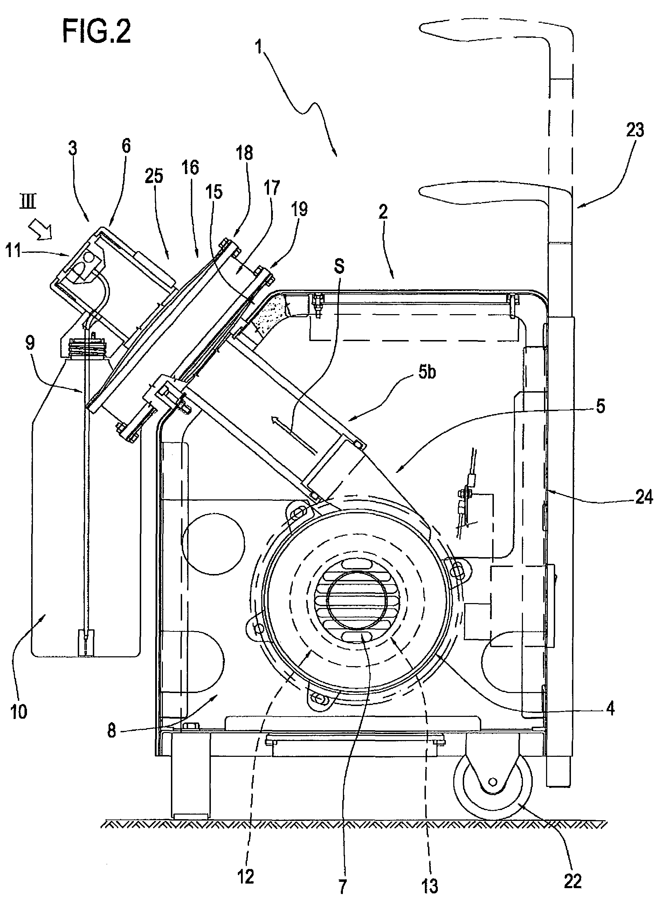

As illustrated in Figure 2, the Venturi effect device 6 is connected by way of an intake pipe 9 to a removable container 10 holding a liquid sanitizing solution.

The Venturi effect device 6 further comprises an end nozzle 11 through which the nebulized liquid solution is diffused in the form of micro-particles into the air external of it.

The nebulizing device 3 also comprises an electric motor 12 for rotationally driving the blowing element 4.

As illustrated in Figure 2, the nebulizing device 3 comprises a second duct 13 for the passage of the air used to cool the electric drive motor 12 of the blowing element 4.

In the proximity of the outlet of the second duct 13 towards the outside, at a second flank 14 of the structure 2, opposite the first flank 8, there is an active carbon filter, not illustrated in the drawing, designed to hold back impurities from the rotating parts of the motor 12, such as for example particles of carbon or copper that wear off the brushes of the motor 12 itself.

The first duct 5 and the second duct 13 are distinct and separate and substantially isolated from each other.

Advantageously, the fact that the first and second ducts 5, 13 are distinct and separate from each other, combined with the presence of a filter on the second duct 13, greatly reduces, or even eliminates, the risk of the nebulizing device 3 contaminating the room air.

That also reduces the risk that the air used to cool the electric motor 12 can alter the temperature and flow conditions of the air which is forced towards the nebulizing device 3 and which thus remains at a temperature substantially corresponding to the temperature of the room where the room sanitizing device is located.

Experimental tests have shown that this feature, that is, the fact that the first duct 5 and the second duct 13 are separate, gives very good results in terms of apparatus efficiency and speed of sanitizing action.

With reference to the accompanying drawings, the apparatus 1 may comprise two ventilation units 16 fixed to an inclined front wall 15 of the structure 2 and adapted to produce a forced circulation of the air inside the room where the apparatus 1 itself is located.

In the preferred, non-limiting embodiment of the invention illustrated, the two ventilation units 16 are located on opposite sides of the Venturi effect device 6 rising up from the inclined front wall 15.

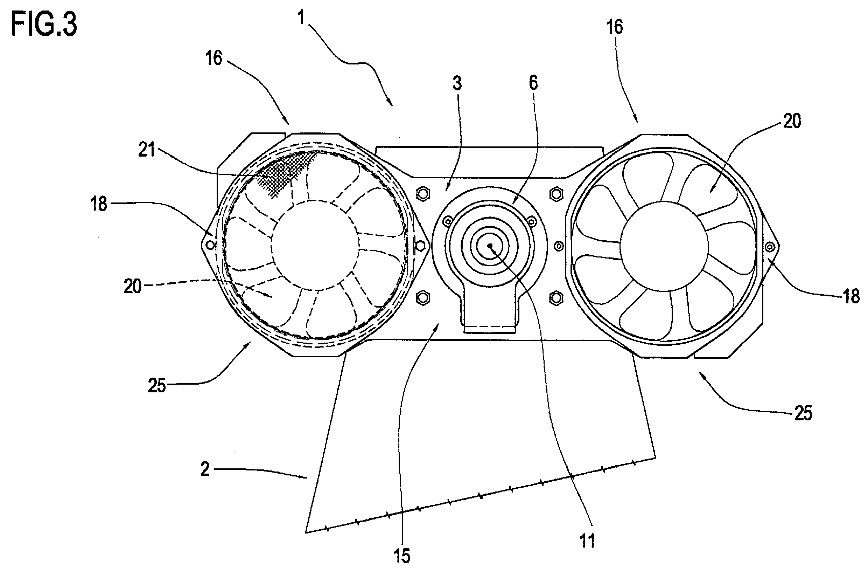

Each ventilation unit 16, substantially cylindrical in shape, comprises a central body 17 enclosed between two flanges 18, 19, an upper one and a lower one, and houses a respective fan 20, shown in Figure 3.

Each ventilation unit 16 comprises an electric motor, not illustrated in the accompanying drawings, for driving the fan 20 and located at the bottom of the ventilation unit 16 itself and rising up therefrom.

The upper flange 18 of each ventilation unit 16 mounts a protective grille 21, only partly illustrated in Figures 1 and 3.

The grille 21 is advantageously made of stainless steel.

The ventilation units 16 in their entirety constitute room air forced circulation means 25.

As illustrated in Figures 1 and 2, the central structure 2 is supported by at least two castors 22 designed to facilitate movement of the apparatus 1, thanks also to an extensible handle 23 fixed to a rear wall 24 of the structure 2 itself.

Each ventilation unit 16 also comprises filtration means, not illustrated, housed in the respective central body 17 and designed to filter the air moved by the circulation means 25 to remove polluting and other unwanted substances from the air.

Depending on the particular substance to be removed or held back, the above mentioned filtration means may comprise filters of different shapes and/or materials.

For example, the filtration means advantageously comprise an active carbon filter for holding back volatile organic substances, ammonium quaternary salts, sodium hypochlorite, phenols, glutaraldehyde, chlorhexidine and ozone.

To remove dust from the air, the filtration means may advantageously comprise a filter made of polymeric material, non-woven fabric and/or cellulose.

To remove silver traces from the air, the filtration means may advantageously comprise a filter containing alkali halides.

To remove hydrogen peroxide and peracetic acid from the air, the filtration means may advantageously comprise a filter containing manganese dioxide or transition metals (such as, for example, Ag, Cu, Fe, Ni, etc.).

The central body 17 of each ventilation unit 16 defines an element for containing the fan 20 and the air filtration means not illustrated.

In use, the apparatus 1 is positioned inside a room to be sanitized.

After the step of nebulizing the liquid sanitizing substance in a manner well known and hence not described in further detail here, a step of forced circulation of the room air is activated through the above mentioned means 25.

More specifically, a control and drive unit, not illustrated, sets the ventilation units 16 in operation for a certain length of time, depending on preset parameters and/or as a function of the measured concentration of the liquid sanitizing substance in the air.

Setting the ventilation units 16 in operation produces the desired forced circulation of the air and at the same time the air is also filtered through the filtration means housed in the ventilation units 16 themselves.

In other words, the air in the room is forced by the ventilation units 16 to circulate through the filtration means and, as it flows through these, the polluting and unwanted substances it contains are removed or reduced in concentration.

Further, according to another important aspect of this invention, as already stressed above, the first duict 5 and the second duct 13 are distinct and separate from each other, thus greatly reducing the risk of the air being contaminated by particles that wear off the moving parts of the nebulizing device 3. Yet another important aspect connected with the ducts 5 and 13 being separate and distinct from each other lies in the fact that the air which circulates through the first duct 5 is not subjected to any heating action because it does not come into direct contact with the electric motor 12.

This aspect, that is to say, the fact that the air used for nebulizing the liquid substance remains at substantially the same temperature as the room, means that the droplets formed are also delivered at the temperature of the room, thereby minimizing oxidation and corrosion of material surfaces in the room, which is a particularly strongly felt problem with prior art nebulizing devices, which do not operate at room temperature.

The apparatus according to the invention achieves the preset aims by allowing a quick and effective reduction of the airborne concentration of toxic and/or polluting and/or other unwanted substances, which in turn means that rooms subjected to sanitizing treatment can be made accessible sooner after treatment.

Obviously, reduction of the airborne concentration of toxic and/or polluting and/or other unwanted substances regards not only nebulized substances resulting from the sanitizing treatment but also unwanted substances already present in the air prior to sanitization.

Advantageously, according to variants of use not illustrated in the accompanying drawings, the apparatus according to the invention can be integrated in air conditioning systems, thus guaranteeing that the conditioned air diffused by such systems is suitably sanitized.

Advantageously, according to yet other variants of use, the apparatus according to the invention can be used in rooms other than those used for strictly medical purposes, for example, offices open to the public, waiting rooms, trains, or aircraft and watercraft interiors.

## Claims

1. A room sanitizing apparatus comprising a device (3) for nebulizing a sanitizing liquid solution, the device (3) comprising a mounting and containment frame (2) and a blowing element (4) which produces an air flow for nebulizing the liquid substance and electric motor means (12) for rotationally driving the blowing element (4), the nebulizing device (3) comprising a first duct (5) for circulating the room air to and from the blowing element (4) and a second duct (13) for the air which cools the electric motor means (12), the first and the second duct (5, 13) being separate and isolated from each other, the apparatus further comprising means (25) for the forced circulation of room air, which are separate from, and independent and external of, the nebulizing device (3), **characterized in that** it comprises filtration means associated with the air circulation means (25) and used for filtering the air and remove or reduce the concentration of polluting and unwanted substances in the air, and **in that** the forced air circulation means (25) are mounted on said frame (2) of the nebulizing device (3).

2. The apparatus according to claim 1, **characterized in that** the forced air circulation means (25) comprise at least one ventilation unit (16).

3. The apparatus according to claim 2, where the ventilation unit (16) comprises a fan (20) and an electric motor for driving the fan (20), **characterized in that** the forced air circulation means (25) comprise an element (17) for containing the fan (20) and the filtration means.

4. The apparatus according to claim 3, **characterized in that** the element (17) for containing the fan (20) and the filtration means is cylindrical in shape and has a protective grille (21).

5. The apparatus according to any of the claims from 2 to 4, **characterized in that** the forced air circulation means (25) comprise two or more ventilation units (16) mounted in parallel.

6. The apparatus according to any of the foregoing claims from 1 to 5, **characterized in that** the filtration means comprise an active carbon filter.

7. The apparatus according to any of the foregoing claims from 1 to 6, **characterized in that** the filtration means comprise an alkali halide filter.

## Patentansprüche

1. Raumdesinfektionsvorrichtung, umfassend eine Einrichtung (3) zum Zerstäuben einer flüssigen Desinfektionslösung, wobei die Einrichtung (3) einen Montage- und Einhausungsrahmen (2) und ein Blaselement (4) umfasst, das einen Luftstrom zur Zerstäubung der flüssigen Substanz erzeugt, sowie elektrische Motormittel (12) zum drehenden Antrieb des Blaselements (4), wobei die Zerstäubungseinrichtung (3) eine erste Leitung (5) für die Umwälzung der Raumluft zum und vom Blaselement (4) und eine zweite Leitung (13) für die Luft, die die elektrischen Motormittel (12) kühlt, umfasst, wobei die erste und die zweite Leitung (5, 13) separat und voneinander isoliert sind und die Vorrichtung zudem Mittel (25) zur Zwangsumwälzung der Raumluft umfasst, die separat und unabhängig und außenseitig von der Zerstäubungseinrichtung (3) angeordnet sind, **dadurch gekennzeichnet, dass** sie Filtermittel umfasst, die mit den Luftumwälzungsmitteln (25) verbunden sind und eingesetzt werden, um die Lift zu filtern und die Konzentration an Schadstoffen und unerwünschten Substanzen in der Luft zu beseitigen oder zu reduzieren, und dadurch, dass die Mittel zur Zwangsumwälzung der Luft (25) am Rahmen (2) der Zerstäubungseinrichtung (3) montiert sind.

2. Vorrichtung nach Anspruch 1, **dadurch gekennzeichnet, dass** die Mittel zur Zwangsumwälzung der Luft (25) mindestens eine Belüftungseinheit (16) umfassen.

3. Vorrichtung nach Anspruch 2, wobei die Belüftungseinheit (16) einen Lüfter (20) und einen Elektromotor für den Antrieb des Lüfters (20) umfasst, **dadurch gekennzeichnet, dass** die Mittel zur Zwangsumwälzung der Luft (25) ein Element (17) zum Enthalten des Lüfters (20) und der Filtermittel umfassen.

4. Vorrichtung nach Anspruch 3, **dadurch gekennzeichnet, dass** das Element (17) zum Enthalten des Lüfters (20) und der Filtermittel eine zylindrische Form und ein Schutzgitter (21) aufweist.

5. Vorrichtung nach einem der Ansprüche 2 bis 4, **dadurch gekennzeichnet, dass** die Mittel zur Zwangsumwälzung der Luft (25) zwei oder mehr Belüftungseinheiten (16) umfassen, die parallel montiert sind.

6. Vorrichtung nach einem der vorhergehenden Ansprüche 1 bis 5, **dadurch gekennzeichnet, dass** die Filtermittel einen Aktivkohlefilter umfassen.

7. Vorrichtung nach einem der vorhergehenden Ansprüche 1 bis 6, **dadurch gekennzeichnet, dass** die Filtermittel einen Alkalihalid-Filter umfassen.

## Revendications

1. Appareil de désinfection de locaux comprenant un dispositif (3) destiné à vaporiser une solution liquide désinfectante, le dispositif (3) comprenant un support de montage et de contenance (2) ainsi qu'un élément de soufflage (4) qui produit un flux d'air pour vaporiser la substance liquide et des moyens motorisés électriques (12) pour entraîner en rotation l'élément de soufflage (4), le dispositif de vaporisation (3) comprenant un premier conduit (5) de circulation de l'air ambiant vers et depuis l'élément de soufflage (4) et un second conduit (13) pour l'air qui refroidit les moyens motorisés électriques (12), le premier et le second conduit (5, 13) étant séparés et isolés l'un de l'autre, l'appareil comprenant de plus des moyens (25) pour la circulation forcée de l'air ambiant, étant séparés et indépendants du, et extérieurs au dispositif de vaporisation (3), **caractérisé en ce qu'**il comprend des moyens de filtration associés aux moyens de circulation d'air (25) et utilisés pour filtrer l'air et supprimer ou réduire la concentration de substances polluantes et indésirables dans l'air, et **en ce que** les moyens de circulation d'air forcée (25) sont montés sur ledit support (2) du dispositif de vaporisation (3).

2. Appareil selon la revendication 1, **caractérisé en ce que** les moyens de circulation d'air forcé (25) comprennent au moins une unité de ventilation (16).

3. Appareil selon la revendication 2, dans lequel l'unité de ventilation (16) comprend un ventilateur (20) et un moteur électrique pour entraîner le ventilateur (20), **caractérisé en ce que** les moyens de circulation d'air forcé (25) comprennent un élément (17) destiné à contenir le ventilateur (20) et les moyens de filtration.

4. Appareil selon la revendication 3, **caractérisé en ce que** l'élément (17) destiné à contenir le ventilateur (20) et les moyens de filtration a une forme cylindrique et possède une grille de protection (21).

5. Appareil selon l'une quelconque des revendications de 2 à 4, **caractérisé en ce que** les moyens de circulation d'air forcé (25) comprennent deux ou plusieurs unités de ventilation (16) montées en parallèle.

6. Appareil selon l'une quelconque des revendications précédentes de 1 à 5, **caractérisé en ce que** les moyens de filtration comprennent un filtre à charbon actif.

7. Appareil selon l'une quelconque des revendications précédentes de 1 à 6, **caractérisé en ce que** les moyens de filtration comprennent un filtre contenant des halogénures alcalins.
